# EUROPÄISCHE PATENTSCHRIFT

(11) **EP 0 625 135 B1**
(45) Veröffentlichungstag und Bekanntmachung des Hinweises auf die Patenterteilung: **02.04.1997**
(21) Anmeldenummer: 93917372.0
(22) Anmeldetag: 27.01.1993
(51) Int. Cl.: C07C 29/76, C07C 31/22, B01D 61/14

(54) **VERFAHREN ZUM REINIGEN VON GLYCERINWASSER**
METHOD OF PURIFYING GLYCERIN WATER
PROCEDE POUR PURIFIER DE L'EAU GLYCERIQUE

(30) Priorität: 05.02.1992 DE 4203157
(43) Veröffentlichungstag der Anmeldung: 23.11.1994
(73) Patentinhaber: Henkel Kommanditgesellschaft auf Aktien, 40191 Düsseldorf (DE)
(72) Erfinder: JEROMIN, Lutz, D-4010 Hilden (DE); JOHANNISBAUER, Wilhelm, D-4006 Erkrath 1 (DE); BLUM, Stefan, D-4018 Langenfeld (DE); SEDELIES, Reinhold, D-6707 Schifferstadt (DE); MOORMANN, Heinrich, D-40591 Düsseldorf (DE); HOLFOTH, Bernd, D-40764 Langenfeld (DE); PLACHENKA, Jürgen, D-4020 Mettmann 2 (DE)
(86) Internationale Anmeldenummer: EP9300184
(87) Internationale Veröffentlichungsnummer: WO9316025

(56) Entgegenhaltungen:
- EP-A- 0 358 255
- EP-A- 0 451 721
- CH-A- 547 765
- FR-A- 2 263 996
- DATABASE WPIL Derwent Publications Ltd., London, GB; AN 86-283915 & SU-A-1 216 176 (SYNTH RUBBER RES VINNITSK OIL-FATS COMPLEX) 7. März 1986

## Beschreibung

Die Erfindung betrifft ein kontinuierliches Verfahren zum Reinigen von Glycerinwasser oleochemischer Herkunft, insbesondere von bei der Hochdruckspaltung natürlicher Fette und Öle mit Dampf in Fettsäure und Glycerin anfallendem Glycerinwasser (Süßwasser).

Zur Herstellung von Glycerin werden natürliche Öle und Fette mit Wasser unter Drücken bis 100 bar und Temperaturen zwischen 100 und 250°C hydrolysiert. Man erhält eine leichte, die Fettsäuren enthaltende und eine schwere Phase, die im wesentlichen aus Glycerin und Wasser besteht und "Süßwasser" genannt wird.

Das Bedürfnis nach möglichst reinem Glycerin erfordert die Entfernung der in den natürlichen Fetten und Ölen sowie dem Süßwasser enthaltenden Verunreinigungen. Diese sind in den nachwachsenden Fetten und Ölen schon von Natur aus vorhanden, weitere Verunreinigungen kommen jedoch bei der Gewinnung (Ernte, Schlachthof) hinzu. Vor der Hydrolyse erfolgt daher eine aufwendige Öl- bzw. Fettreinigung.

Nach der Hochdruckspaltung der Öle und Fette in Glycerin und Fettsäuren erhält man ein Zweiphasengemisch mit einer schweren Glycerin/Wasser-Phase und einer leichten Fettsäurephase, die aus dem Gemisch durch Schwerkraftabscheidung abgetrennt wird. Dabei kann jedoch nicht verhindert werden, daß geringe Mengen an Fettsäuren und anderen Fettstoffen mit der Glycerinwasserphase abgezogen werden. Auch die sich bei der Spaltung außerdem bildenden geringen Mengen von Nebenprodukten, z. B. Di- und Monoglyceride, sowie Triglyceride sind im Süßwasser enthalten. Die Verunreinigungen im Glycerinwasser werden unter dem Sammelbegriff MONG (Matter of Organic Non Glycerol) zusammengefaßt.

Nach der Phasentrennung wird die Glycerinwasserphase auf Normaldruck entspannt. Dabei werden die organischen Verunreinigungen stark emulgiert. Die Emulsion wird durch Eindampfen zu 75 bis 85 %igem Glycerinwasser aufkonzentriert und dann zu Reinglycerin aufbereitet. Die Verunreinigungen führen bei der Eindampfung zu Verkrustungen auf den Wärmeaustauscherflächen und daher zu beträchtlichen Leistungseinbußen. Die durch die größeren Verweilzeiten bedingte Kapazitätseinbuße beim Eindampfen, die Produktverluste und das Erfordernis weiterer Reinigungsschritte sind weitere Nachteile im Stand der Technik.

Eine Zusammenstellung der bekannten Verfahren zur Herstellung von Reinglycerin aus Glycerinwasser (Süßwasser) findet sich in J. Am. Oil Chemists Soc., 1979, S. 812 A-819 A.

Problematisch bei der Reinigung des Glycerinwassers sind insbesondere zwei Umstände. Zum einen müssen kontinuierlich große Produktmengenströme, die 20 m³/h übersteigen, verarbeitet werden. Zum anderen ändert sich in der großtechnischen Praxis das für die Fettspaltung eingesetzte Produkt häufig. So werden sowohl Palm-, Palmkern-Öl, Rindertalg, Rüböl, Fischöl und andere Öle und Fette verarbeitet. Die Verunreinigungen im Glycerinwasser spiegeln die C-Kettenverteilung der Einsatzprodukte wieder, so daß z. B. eine Filtration wegen der unterschiedlichen Stockpunkte der emulgierten MONG-Phase nicht kontinuierlich durchführbar ist.

Ein Verfahren zum Reinigung von Glycerinwasser wird in der EP-A-0 358 255 beschrieben. Eingesetzt werden keramische Membranen. Dabei kann es bei einem häufigen Wechsel der Herkunft des Glycerinwassers und damit bei Verunreinigungen der unterschiedlichsten Art zu einer Verblockung der Membran kommen, wenn das rohe Glycerinwasser ohne weitere Vorbehandlung dem Membranfiltrationsmodul zugeleitet wird.

Der Erfindung liegt daher die Aufgabe zugrunde, ein besonders wirtschaftliches Verfahren der eingangs genannten Art bereitzustellen, das große Produktmengenströme von Glycerinwasser bei häufigem Wechsel der Herkunft kontinuierlich, effektiv und wirtschaftlich zu reinigen in der Lage ist.

Diese Aufgabe wird erfindungsgemäß dadurch gelöst, daß man aus der wäßrigen Phase mittels eines Plattenphasentrenners Fettstoffe mit Durchmessern oberhalb von 100 µm abtrennt und diese in die Hochdruckspaltung zurückführt, die derart vorgereinigte wäßrige Phase im Querstrom über eine Filtrationsmembran leitet und das Konzentrat zum Eingang des Plattenphasentrenners zurückführt. Vorzugsweise ist die Filtrationsmembran als Mikrofiltrationsmembran ausgebildet. Man erhält ein Permeat, das in nachfolgenden Schritten durch Eindampfen aufkonzentriert wird.

Erfindungsgemäß wird das Glycerinwasser in zwei Stufen mit Hilfe von durchströmten Apparaten ohne bewegliche Teile gereinigt. Die Eigenart der Querstrom-Membranfiltration, nämlich eine Belagbildung auf der Membranoberfläche durch von der überströmenden Flüssigkeit ausgeübte Scherkräfte zu verringern, ist hier wegen des MONG-Anteils sehr wichtig, da dieser zum Verblocken der Membran führen kann.

Besonders vorteilhaft für den kontinuierlichen Betrieb ist es, wenn man die Filtrationsmembran von Zeit zu Zeit durch Rückspülen mit Lauge reinigt. Das an sich bekannte Prinzip der Rückspülung von Membranen wird bisher zum Abbau von Belägen suspendierter Inhaltsstoffe angewendet. Dabei wird auf eine vollständig belegte Membran permeatseitig Druck aufgebracht und auf diese Weise Permeat durch die Membran zurückgedrückt. Der Belag wird abgesprengt und konzentratseitig ausgetragen. Erfindungsgemäß wird dieser Schritt entsprechend angewendet bei den gelartigen, hochviskosen Belägen, die aus MONG bestehen. Bei Beginn der Rückspülung wird der Produktionskreislauf abgesperrt und anschließend von der Permeatseite Lauge, z. B. Natronlauge mit Druck in die Membran gepreßt. Die Beläge werden verseift und damit wasserlöslich.

Vorzugsweise fängt man die verbrauchte Rückspülflüssigkeit in einem Behälter auf, spült die Membran nach dem Rückspülen mit Säure nach, sammelt auch diese Spülflüssigkeit in dem Behälter, so daß dort die gebildeten Seifen gespalten werden, und führt schließlich den Inhalt des Behälters zum Eingang des Plattenphasentrenners zurück.

Insbesondere für einen kontinuierlichen Betrieb ist es vorteilhaft, wenn man den Permeatfluß durch die Membran durch Änderung des transmembranen Drucks auf einen konstanten Wert regelt. Einem Absinken des Permeatstromes kann also durch konzentratseitige Druckerhöhung entgegengewirkt werden. Diese Regelung kann bei eine Deckschicht bildenden Produkten nur so lange betrieben werden, bis eine obere Druckgrenze erreicht ist. Es wird daher vorgeschlagen, daß man die Rückspülung bei Überschreiten eines vorgegebenen transmembranen Druckes beginnt. Die Permeatstromregelung und die Auslösung der Rückspülung kann auf einfache Weise automatisiert werden.

Ein besonders klares, trübungsfreies gereinigtes Glycerinwasser erhält man, wenn man die Membranfiltration bei Temperaturen unter 50°C, insbesondere unter 40°C durchführt.

Vorgeschlagen wird auch, daß man eine rückspülbare Membran, insbesondere eine keramische Membran, vorzugsweise aus Graphit oder Al₂O₃, mit einem Porendurchmesser von 0,5 bis 2 µm einsetzt. Die Membran ist kompakt, hochgradig chemisch und mechanisch belastbar und eignet sich daher besonders für den großtechnischen Einsatz. Die gegensätzlichen Anforderungen, möglichst hohe Permeatflüsse bei gleichzeitig guter MONG-Rückhaltung sowie gutem Reinigungsverhalten zu erreichen, konnten mit den genannten Porendurchmessern erfüllt werden.

Die Rückspülzyklen der Membran lassen sich verlängern, wenn man die vom Plattenphasentrenner vorgereinigte wäßrige Phase durch einen Faserbettabscheider leitet, die erhaltene leichte Phase in die Hochdruckspaltung zurückführt und die schwere Phase der Membranfiltration zuführt, dessen Konzentrat man zum Eingang des Faserbettabscheiders zuleitet.

Sofern Verunreinigungen befürchtet werden, die irreversible Verblokkungen am Faserbettabscheider oder an der Membran verursachen können, schaltet man in einer weiteren vorteilhaften Ausgestaltung der Filtrationsmembran eine Adsorptionseinheit vor. Die Adsorption kann z. B. an Polyethylengranulat erfolgen.

Nachstehend sind Ausführungsbeispiele und Versuchsergebnisse anhand von Zeichnungen näher erläutert. Es zeigen
- Figur 1: ein Schema einer üblichen Anlage zur kontinuierlichen Fettspaltung,
- Figur 2: ein Anlagenschema gemäß einem Ausführungsbeispiel der Erfindung,
- Figur 3: eine schematische Darstellung zur Erläuterung der Wirkungsweise der Membran-Rückspülung,
- Figur 4: eine Darstellung von Versuchsergebnissen,
- Figur 5: eine weitere Darstellung von Versuchsergebnissen,
- Figur 6: ein Anlagenschema zu einer weiteren Ausführungsform der Erfindung und
- Figur 7: ein Anlagenschema zu einer Modifizierung dieser Ausführungsform.

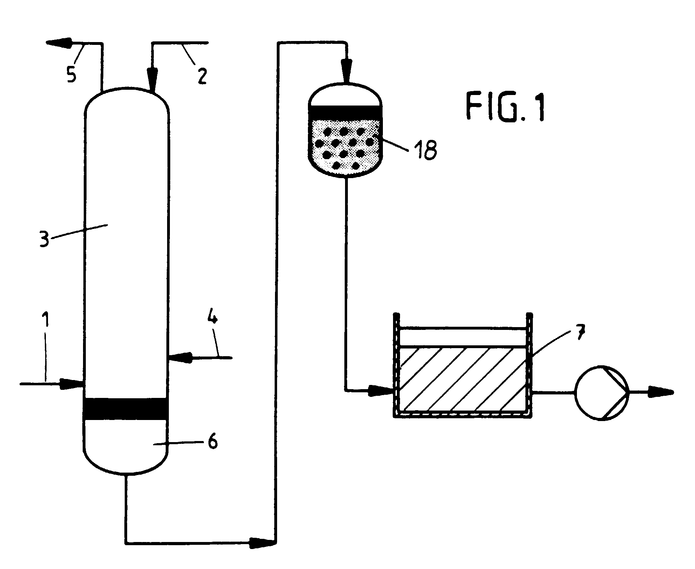

Die zu reinigende glycerinhaltige wäßrige Phase kann z. B. aus einer Anlage zur Hochdruckspaltung natürlicher Fette und Öle (Triglyceride) mit Wasser in Fettsäure und Glycerin erhalten werden. Eine übliche Anlage ist in Figur 1 dargestellt. Fett/Öl 1 und Wasser 2 werden am Fuß bzw. Kopf einer Spaltkolonne 3 eingespeist. Der Reaktor wird mit Hochdruckdampf 4 von etwa 250°C und 75 bar beheizt. Die entstandene Fettsäure 5 wird am Kopf abgezogen. Ein Glycerinwasser-MONG-Gemisch 6 sammelt sich im Sumpf, wird dort abgezogen, im Behälter 18 auf Normaldruck entspannt und in einem Abscheider 7 gesammelt. Die Verunreinigungen (MONG) des Glycerinwassers enthalten zu 80 % freie Fettsäuren, 15 % Fettsäureester und 5 % sonstige Verunreinigungen.

Zur Reinigung des rohen Glycerinwassers wird es nach einer Vorreinigung in einem Plattenphasentrenner einer Membranfiltration unterzogen. Das bei der Membranfiltration anfallende Konzentrat wird zum Eingang des Plattenphasentrenners zurückgeleitet. Von diesem abgetrenntes MONG wird in die Hochdruckspaltung zurückgeführt.

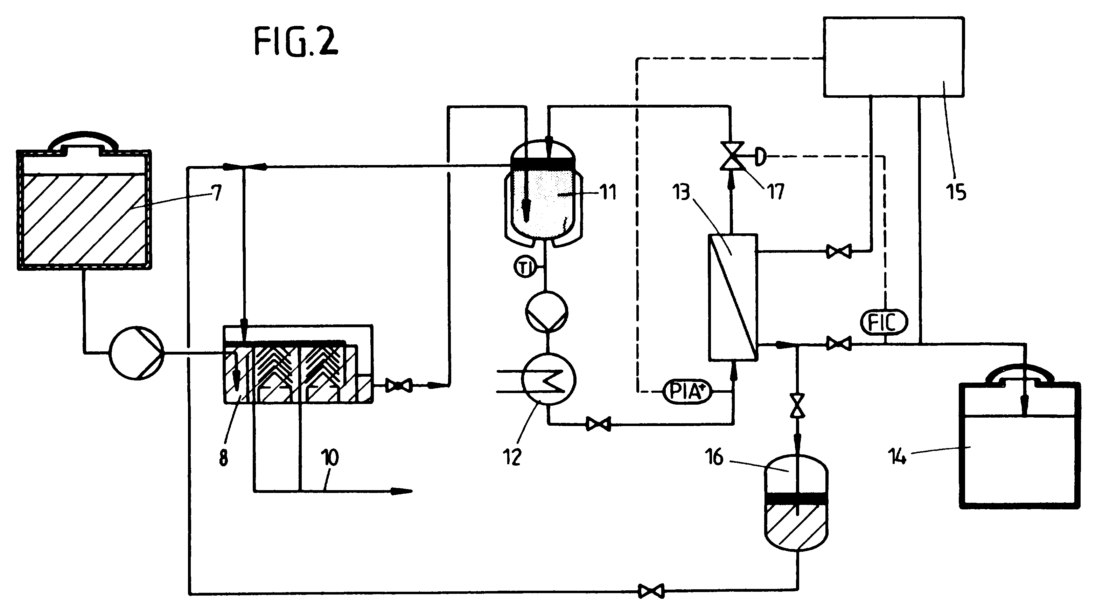

Eine zum Durchführen dieses erfindungsgemäßen Verfahrens geeignete Anlage ist schematisch in Figur 2 dargestellt. Das in einer Vorlage 9 gesammelte verunreinigte Glycerinwasser wird in einen Plattenphasentrenner 8 gepumpt. Die Verunreinigungen (MONG) werden über eine Leitung 10 zur Hochdruckspaltung zurückgeführt, und das vorgereinigte Glycerinwasser fließt in einen Abscheider 11. Dort findet eine erneute Phasentrennung statt, und die leichte Phase wird dem Plattenphasentrenner 8 zugeleitet. Die schwere Phase wird über einen zur Einstellung der Betriebstemperatur der Membranfiltration dienenden Erhitzer/Kühler 12 einem Mikrofiltrationsmodul 13 zugeleitet. Das Konzentrat fließt in den Abscheider 11 zurück, das Permeat wird in einer Vorlage 14 aufgefangen. Zur Rückspülung des Membranmoduls 13 ist ferner eine Regeleinrichtung 15 und ein Behälter 16 zur Seifenspaltung vorgesehen. Einrichtungen zum Regeln des Permeatflußes (FIC, Ventil 17) sowie zum Einstellen des transmembranen Druckes mittels Androsselung des Retentatstromes (PIA) sind in der Anlage ebenfalls enthalten.

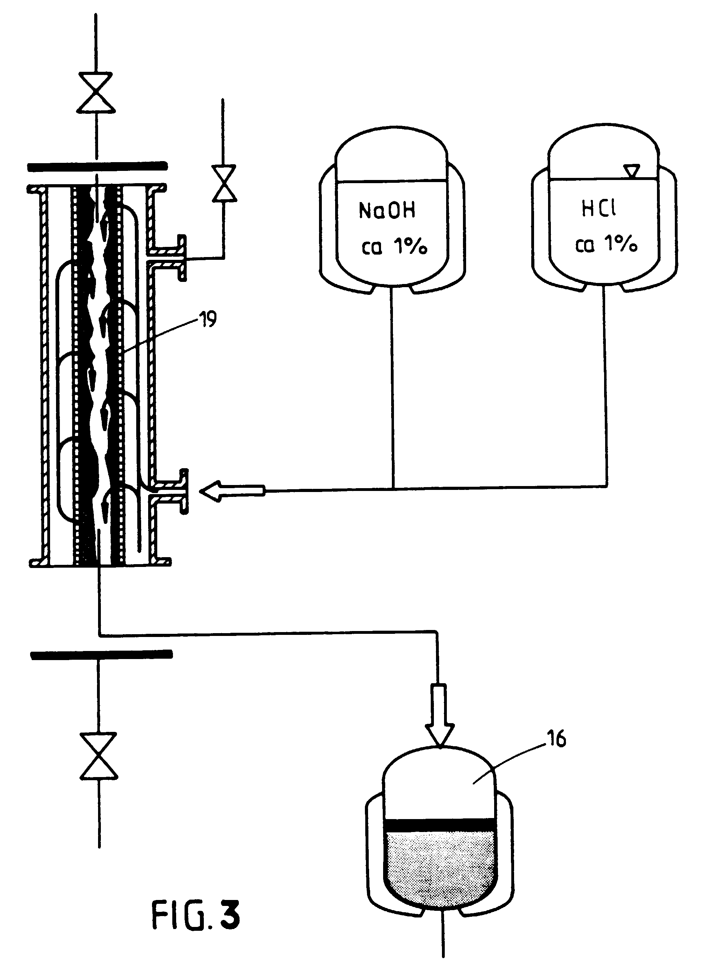

Die Rückspülung des Membranmoduls ist in Figur 3 gezeigt. Die Membran 19 wird permeatseitig zunächst mit Natronlauge und dann mit Säure gespült. Die Spülflüssigkeit mit dem sich von der Innenseite der Membran 19 abgelösten Belag wird in einem Behälter 16 aufgefangen.

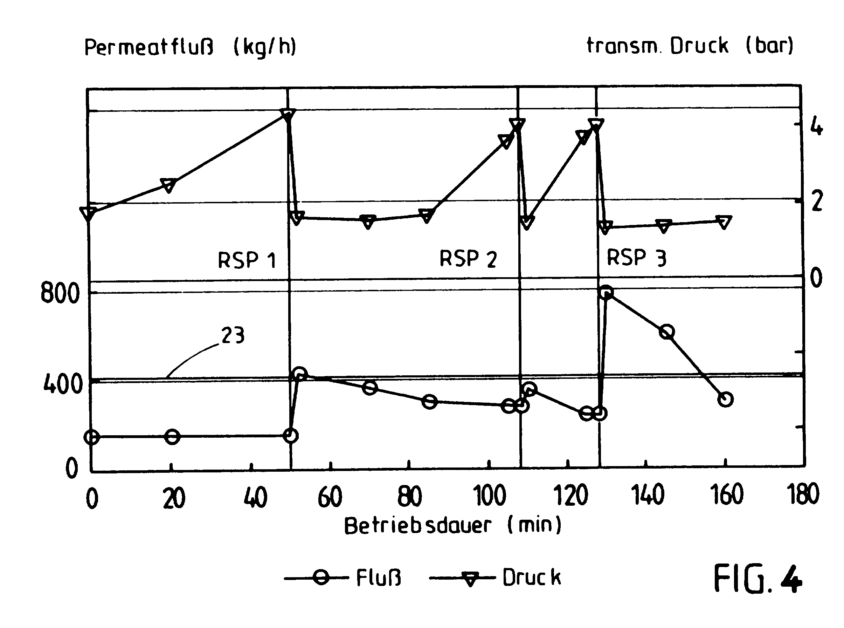

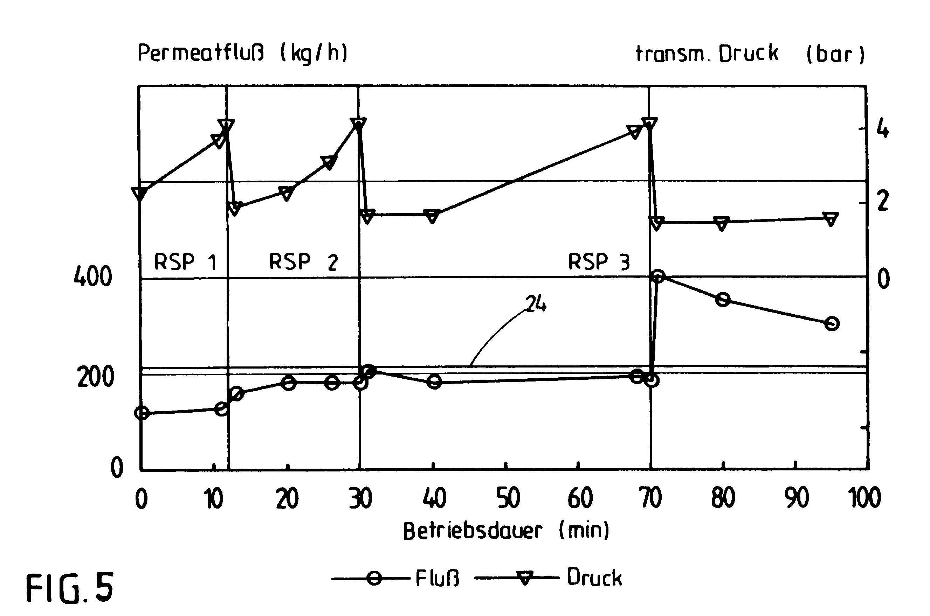

Die Figuren 4 und 5 zeigen Versuchsergebnisse mit dem erfindungsgemäßen Verfahren. Die Versuche wurden mit der in Figur 2 gezeigten Anlage durchgeführt.

### Beispiel I:

Das Ausgangsmaterial war Glycerinwasser mit 15 % Glycerin aus der Hochdruckspaltung. Es war braun und hatte eine trübe Konsistenz. Die in Figur 4 gezeigten Ergebnisse wurden mit einer Al₂O₃-Membran mit 0,8 um Porenweite und einer Membranfläche von 0,2 m² bei einer Temperatur von 70°C erhalten. Linie 23 kennzeichnet den durchschnittlichen Permeatfluß von 414 kg/h entsprechend 2 071 kg/m²h.

Mit RSP sind die Rückspülvorgänge bezeichnet. Bei Überschreitung eines transmembranen Grenzdruckes von 4 bar wurde die Rückspülung ausgelöst. Die Rückspülungsdauer betrug für NaOH (aq. 1 %) sowie für H₂SO₄ (aq. 1 %) je 10 Sekunden. Unmittelbar nach der Rückspülung wurden bei einer transmembranen Druckdifferenz von 1,6 bar Permeatflüsse von bis zu 4 000 l/m²h gemessen, die im Laufe einer halben Stunde auf 1 000 l/m²h abfielen. Durch die Regelung der Rückspülung konnte der Permeatfluß eine weitere halbe Stunde konstant gehalten werden, bis die Rückspülung einsetzte. Anschließend wurden erneut extrem hohe Flüsse gemessen. Auf diese Weise konnte ein mittlerer Permeatfluß von ca. 2 000 l/m²h erreicht werden. Dazu war etwa 1 Rückspülung pro Stunde nötig. Durch die integrierte Seifenspaltung unter Rückführung in den Plattenphasentrenner betrug der Na₂SO₄-Gehalt im Permeat weniger als 50 ppm. Das Permeat war klar und gelb gefärbt, zeigte bei Abkühlung jedoch eine leichte Trübung. Dieser Effekt konnte bei niedrigeren Betriebstemperaturen (32°C) vermieden werden.

### Beispiel II:

Die in Figur 5 gezeigten Werte wurden mit 40 %igem Glycerinwasser aus einer Niederdruckumesterung bei einer Betriebstemperatur von 32°C erhalten. Nach der ersten und zweiten Rückspülung betrug der Sollwert des Permeatflusses 180 kg/h, nach der dritten Rückspülung 300 kg/h. Der mittlere Fluß von 214 kg/h entsprechend 1 070 kg/m²h ist als Linie 24 eingezeichnet. Hier waren durchschnittlich 2 Rückspülungen pro Stunde notwendig.

Es bieten sich zwei Meßgrößen an, um die Rückhaltung der Membran zu beziffern: Partikelgrößenverteilung und Trübung. Als signifikanter Wert für die Partikelgrößenverteilung dient die Partikelgröße x90, die den Durchmesser angibt, der von 90 % der Partikel unterschritten wird.

Die Partikelgröße x90 betrug bei 70°C unabhängig vom Einsatz ungefähr 2,5 µm, bei 30°C war das Permeat partikelfrei.

Die Trübung wurde relativ zu Wasser bestimmt, dabei wurde für Wasser eine Trübung von 0 % angenommen. Das Permeat, das bei 70°C gewonnen wurde, wies eine Trübung von 5 %, das von 30°C von 1,5 % auf, was jedoch auf die gelbe Färbung zurückzuführen ist.

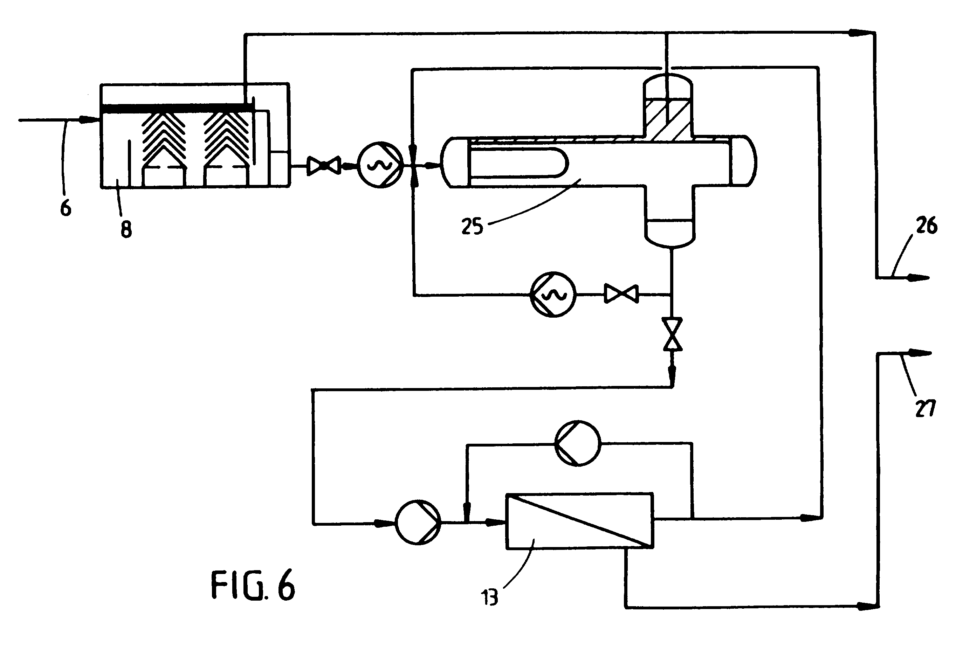

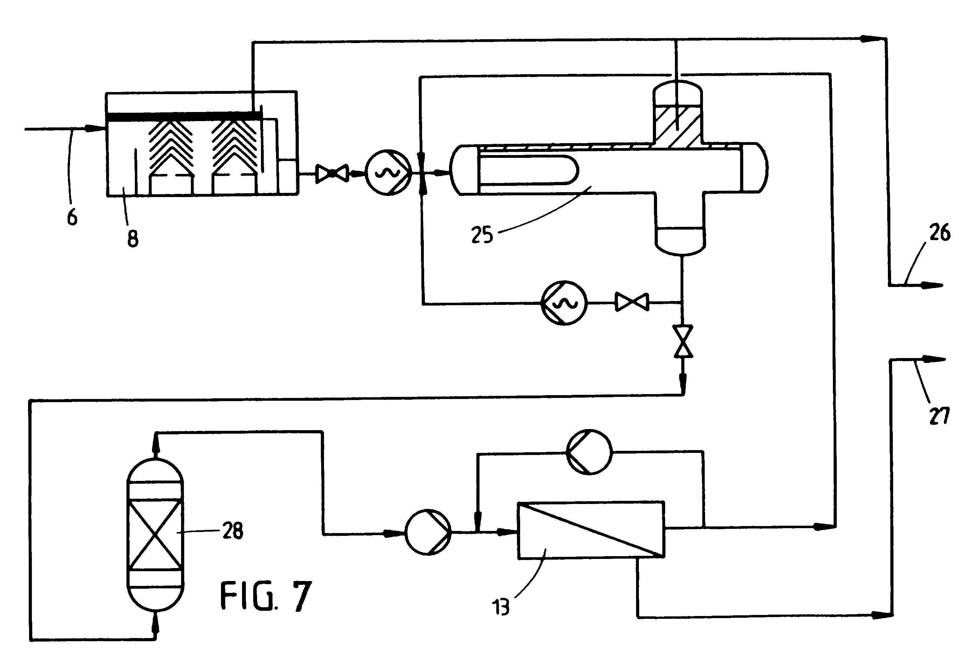

In einer anderen Ausführungsform der Erfindung sind ein Plattenphasentrenner 8, ein Faserbettabscheider 25 sowie eine Mikrofiltrationsmodul 13 in Reihe geschaltet. In den Figuren 6 und 7 sind entsprechende Anlagen dargestellt. Dabei haben auch hier gleiche Bezugszeichen die gleiche Bedeutung. Mit 26 ist MONG, mit 27 das gereinigte Glycerinwasser bezeichnet.

Es handelt sich bei diesem Verfahren um eine stufenweise Spaltung der Emulsion mit Hilfe von durchströmten Apparaten ohne bewegliche Teile. Dabei dient jede Stufe zur Vorreinigung des Folgeprozesses. Im Plattenphasentrenner erfolgt die Abtrennung von Tropfen und Partikeln mit größeren Durchmessern als 100 µm, wobei schon eine kontinuierliche MONG-Phase abgerahmt werden kann. Zusätzlich können grobe, schwere Schmutzpartikel abgetrennt und am Boden abgezogen werden. Die Zwischenphase gelangt in den Faserbettabscheider (Koaleszenzabscheider), dessen zentrale Elemente dicht gewickelte Faserkerzen bilden.

Das Material der Fasern läßt sich optimal an die Produkteigenschaften anpassen, des weiteren kann die Faserstärke variiert werden.

Die Faserwahl hängt von den Produkteigenschaften ab, besonders aber von der Viskosität, die sowohl stark temperaturabhängig ist, als auch für die verschiedenen Produkte (C-Ketten-Länge) deutlich variiert. So kann z. B. eine 5 µm-Kerze bei 70°C bei Glycerinwasser aus der Spaltung pflanzlicher Öle (C8-C12) eine gute Emulsionsspaltung erbringen, zunehmende Anteile an Talg, Rüb- oder Fischöl (C18-C22) führen jedoch bei der gleichen Temperatur zur allmählichen Verstopfung. Durch Anheben der Temperatur kann die Verstopfung wieder rückgängig gemacht werden. Dies wird in einer Druckverluststeuerung ausgenutzt. Hierbei wird der Druckverlust über der Faserkerze als Steuergröße und die Temperatur des zu reinigenden Glycerinwassers als Regelgröße verwendet.

Es ist bekannt, daß die Faserkerzen einen bestimmten Druckverlust für den optimalen Betrieb benötigen. Der Druckverlust läßt sich für jedes Glycerinwasser durch exakte Regelung der Temperatur genau einstellen.

Bei bestimmungsgemäßem Betrieb verlassen den Koaleszenzabscheider 2 Phasen, eine leichtere MONG-Phase und eine gereinigte Glycerinwasser-MONG-Restemulsion, die auch mit feinsten Fasern nicht vollständig gespalten werden kann. Diese Phase wird der Mikrofiltrationsstufe zugeleitet.

Im Falle von Verunreinigungen, die irreversible Verblockungen am Faserbettabscheider 25 oder an der Membran verursachen können (z. B. Polyethylen aus Schlachthofsäcken), kann eine Adsorptionskolonne 28, im o. g. Falle mit Polyethylengranulat gefüllt, zur Adsorption der Verschmutzungen integriert werden (Figur 7).

### Bezugszeichenliste

- 1: Fett/ÖL
- 2: Wasser
- 3: Spaltkolonne
- 4: Hochdruckdampf
- 5: Fettsäure
- 6: Glycerinwasser-MONG-Gemisch
- 7: Abscheider
- 8: Plattenphasentrenner
- 9: Vorlage
- 10: Leitung
- 11: Abscheider
- 12: Erhitzer/Kühler
- 13: Mikrofiltrationsmodul
- 14: Vorlage, Permeattank
- 15: Regeleinrichtung
- 16: Behälter
- 17: Ventil
- 18: Behälter
- 19: Membran
- 23: Linie
- 24: Linie
- 25: Faserbettabscheider
- 26: MONG
- 27: gereinigtes Glycerinwasser
- 28: Adsorptionskolonne

## Patentansprüche

1. Kontinuierliches Verfahren zum Reinigen von Glycerinwasser oleochemischer Herkunft, insbesondere von bei der Hochdruckspaltung natürlicher Fette und Öle mit Dampf in Fettsäure und Glycerin anfallendem Glycerinwasser (Süßwasser),
**dadurch gekennzeichnet**,
daß man aus der wäßrigen Phase mittels eines Plattenphasentrenners Fettstoffe mit Durchmessern oberhalb von 100 µm abtrennt und diese in die Hochdruckspaltung zurückführt, die derart vorgereinigte wäßrige Phase im Querstrom über eine Filtrationsmembran leitet und das Konzentrat zum Eingang des Plattenphasentrenners zurückführt.

2. Verfahren nach Anspruch 1,
**dadurch gekennzeichnet**,
daß die Filtrationsmembran als Mikrofiltrationsmembran ausgebildet ist.

3. Verfahren nach Anspruch 1 oder 2,
**dadurch gekennzeichnet**,
daß man die Filtrationsmembran von Zeit zu Zeit durch Rückspülen mit Lauge reinigt.

4. Verfahren nach Anspruch 3,
**dadurch gekennzeichnet**,
daß man die verbrauchte Rückspülflüssigkeit in einem Behälter auffängt, die Membran nach dem Rückspülen mit Säure nachspült, auch diese Spülflüssigkeit in dem Behälter sammelt, so daß dort die gebildeten Seifen gespalten werden, und schließlich den Inhalt des Behälters zum Eingang des Plattenphasentrenners zurückführt.

5. Verfahren nach einem der Ansprüche 1 bis 4,
**dadurch gekennzeichnet**,
daß man den Permeatfluß durch die Membran durch Änderung des transmembranen Drucks auf einen konstanten Wert regelt.

6. Verfahren nach Anspruch 5,
**dadurch gekennzeichnet**,
daß man die Rückspülung bei Überschreiten eines vorgegebenen transmembranen Drucks beginnt.

7. Verfahren nach einem der Ansprüche 1 bis 6,
**dadurch gekennzeichnet,**
daß man eine rückspülbare Membran, insbesondere eine keramische Membran, vorzugsweise aus Graphit oder Al₂O₃, mit einem Porendurchmesser von 0,5 bis 2 µm einsetzt.

8. Verfahren nach einem der Ansprüche 1 bis 7,
**dadurch gekennzeichnet**,
daß man die vom Plattenphasentrenner vorgereinigte wäßrige Phase durch einem Faserbettabscheider leitet, die erhaltene leichte Phase in die Hochdruckspaltung zurückführt und die schwere Phase der Membranfiltration zuführt, dessen Konzentrat man zum Eingang des Faserbettabscheiders zuleitet.

9. Verfahren nach Anspruch 8,
**dadurch gekennzeichnet**,
daß man den Druckverlust über dem Faserbettabscheider durch kontinuierliches Einstellen der Temperatur des zufließenden Glycerinwassers konstant hält.

10. Verfahren nach einem der Ansprüche 1 bis 9,
**dadurch gekennzeichnet**,
daß man der Filtrationsmembran eine Adsorptionseinheit vorschaltet.

## Revendications

1. Procédé en continu d'épuration d'eau glycérinée d'origine oléochimique notamment d'eau glycérinée obtenue par séparation sous haute pression de graisses et huiles naturelles avec de la vapeur en acide gras et glycérine (eau douce),
caractérisé en ce qu'on
on sépare de la phase aqueuse au moyen d'un séparateur de phases à plaques les solides ayant des diamètres supérieurs à 100 µm et on les recycle dans la séparation sous haute pression, on conduit ainsi la phase aqueuse pré-épurée en sens contraire d'une membrane à filtration et recycle le concentré à l'entrée du séparateur de phases à plaques.

2. Procédé selon la revendication 1,
caractérisé en ce que
la membrane à filtration est constituée en membrane de microfiltration.

3. Procédé selon la revendication 1 ou 2,
caractérisé en ce qu'
on purifie la membrane à filtration de temps en temps par balayage en sens inverse avec une lessive alcaline.

4. Procédé selon la revendication 3,
caractérisé en ce qu'
on recueille dans une cuve le liquide usé de balayage en sens inverse, on rince la membrane après balayage en sens inverse avec de l'acide, on recueille aussi ce liquide de rinçage dans la cuve, de sorte que les savons qui y sont formés sont décomposés, et enfin on recycle le contenu de la cuve à l'entrée du séparateur de phases à plaques.

5. Procédé selon l'une des revendications 1 à 4,
caractérisé en ce qu'
on régule le flux du perméat à travers la membrane à une valeur constante par modification de la pression transmembranique.

6. Procédé selon la revendication 5,
caractérisé en ce qu'
on commence le balayage en sens inverse en dépassant une pression donnée transmembranique.

7. Procédé selon l'une des revendications 1 à 6,
caractérisé en ce qu'
on utilise une membrane balayable en sens inverse, notamment une membrane céramique, de préférence en graphite ou Al₂O₃, avec un diamètre de pore de 0,5 à 2 µm.

8. Procédé selon l'une des revendications 1 à 7,
caractérisé en ce qu'
on conduit la phase aqueuse pré-épurée par le séparateur de phase à plaques à travers un séparateur à lit de fibres, on recycle la phase légère obtenue dans la séparation sous haute pression et on envoie la phase lourde à la filtration par membrane, dont on dirige le concentré à l'entrée du séparateur à lit de fibres.

9. Procédé selon la revendication 8,
caractérisé en ce qu'
on maintient constante la perte de charge dans le séparateur à lit de fibres par réglage en continu de la température de l'eau glycérinée qui doit s'écouler.

10. Procédé selon l'une des revendications 1 à 9,
caractérisé en ce qu'
on place une unité d'adsorption en amont de la membrane de filtration.

## Claims

1. A continuous process for the purification of glycerol water of oleochemical origin, more particularly glycerol water (sweet water) accumulating in the high-pressure hydrolysis of natural fats and oils with steam into fatty acid and glycerol, characterized in that fatty compounds larger than 100 µm in diameter are removed from the aqueous phase by means of a plate-type phase separator and are returned to the high-pressure hydrolysis process, the aqueous phase thus prepurified is passed through a crossflow filtration membrane and the concentrate is returned to the entrance of the plate-type phase separator.

2. A process as claimed in claim 1, characterized in that the filtration membrane is a microfiltration membrane.

3. A process as claimed in claim 1 or 2, characterized in that the filtration membrane is periodically cleaned by backwashing with alkali hydroxide.

4. A process as claimed in claim 3, characterized in that the used backwashing liquid is collected in a vessel, the membrane is rinsed with acid after backwashing, the rinsing liquid is also collected in the vessel so that the soaps formed therein are hydrolyzed and, finally, the contents of the vessel are returned to the entrance of the plate-type phase separator.

5. A process as claimed in any of claims 1 to 4, characterized in that the flow of permeate through the membrane is kept constant by changing the transmembranal pressure.

6. A process as claimed in claim 5, characterized in that backwashing begins when a predetermined transmembranal pressure is exceeded.

7. A process as claimed in any of claims 1 to 6, characterized in that a back-washable membrane, more especially a ceramic membrane, preferably of graphite or Al₂O₃, having a pore diameter of 0.5 to 2 µm is used.

8. A process as claimed in any of claims 1 to 7, characterized in that the aqueous phase prepurified in the plate-type phase separator is passed through a fibre bed separator, the light phase obtained is returned to the high pressure hydrolysis process and the heavy phase is fed to the membrane filtration stage of which the concentrate is delivered to the entrance of the fibre bed separator.

9. A process as claimed in claim 8, characterized in that the pressure loss through the fibre bed separator is kept constant by continuous adjustment of the temperature of the inflowing glycerol water.

10. A process as claimed in any of claims 1 to 9, characterized in that the filtration membrane is preceded by an adsorption unit.
